# EUROPEAN PATENT APPLICATION

(11) **EP 4 191 610 A1**
(43) Date of publication of application: **07.06.2023**
(21) Application number: 20947152.3
(22) Date of filing: 29.09.2020
(51) Int. Cl.: G16H 50/30, G06Q 10/06, G06T 7/00, G06T 7/62, G01N 33/68

(54) **APPLICATION OF HBP IN PROGNOSIS AND RISK WARNING FOR COVID-19 PATIENT**

(30) Priority: 30.07.2020 CN 202010750016
(71) Applicant: Joinstar Biomedical Technology Co., Ltd., Hangzhou, Zhejiang 311188 (CN)
(72) Inventor: SUN, Baoqing, Hangzhou, Zhejiang 311188 (CN); ZHONG, Nanshan, Hangzhou, Zhejiang 311188 (CN); ZHOU, Xuyi, Hangzhou, Zhejiang 311188 (CN); XUE, Mingshan, Hangzhou, Zhejiang 311188 (CN); HONG, Longbin, Hangzhou, Zhejiang 311188 (CN); DONG, Ming, Hangzhou, Zhejiang 311188 (CN)
(74) Representative: dompatent von Kreisler Selting Werner - Partnerschaft von Patent- und Rechtsanwälten mbB
(86) International application number: PCT/CN2020/118688
(87) International publication number: WO 2022/021597

(57) **Abstract**

Provided are a method for predicting a risk of deterioration of a pathogenic condition of a patient suffering a coronavirus disease (COVID-19), especially a severe patient suffering a COVID-19 and a kit thereof. The method of the present application may effectively predict the deterioration of the pathogenic condition of the patient suffering a COVID-19, thereby carrying out clinical intervention in advance, to improve prognosis of the patient suffering a COVID-19. The present application finds for the first time that the level of a heparin-binding protein (HBP) can be used as a significant indicator for early predicting relapse of the patient suffering a COVID-19, expression time of an HBP indicator is around 5 days earlier than that of a series of other clinical indicators, and thus the HBP indicator particularly has a clinical value.

## Description

### TECHNICAL FIELD

The present application relates to early warning of a prognostic risk of a patient suffering a coronavirus disease (COVID-19).

### BACKGROUND

The pathogenesis of a coronavirus disease (COVID-19) is being gradually clarified at present, and clinicians have begun to pay attention to rehabilitation and treatment of sequelae of the COVID-19. Respiratory symptoms are the main manifestation of the COVID-19, and severe diffuse alveolar epithelial damage is one of the main reasons leading to death. Especially in severe patients, pulmonary exudation are obviously increased, and abnormal dilation of pulmonary vessels at a lesion site can be observed. However, organs other than lungs, such as liver, kidney and heart, may also suffer direct or secondary damage.

A wide range of studies have reported that some positive patients suffering a COVID-19 are negative for viruses after treatment, but pathogenic conditions are suddenly deteriorated, and the patients can even die in a viral remission period, which can be caused by false negative or potential cardiovascular diseases aggravated by infection. On the other hand, an inflammatory state caused by the COVID-19 may persist after viral ribonucleic acid (RNA) is no longer tested, and secondary inflammatory storms can occur in the remission period. Therefore, timely intervention before pathogenic conditions of viral negative patients suffering a COVID-19 are deteriorated can significantly improve prognosis. In this regard, it will be extremely valuable to identify biomarkers that predict which patients in the remission period will relapse.

### SUMMARY

A heparin-binding protein (HBP), also known as azurocidin or cationic antimicrobial protein of 37 KDa (CAP37), is a secreted granulin located in a polymorphonuclearleukocyte (PMN) secretory vesicle and an azurophilic granule. A sequence of the HBP is publicly obtainable. For example, the sequence of the HBP is obtained by an accession number NP 001691 REGION: 27 .. 248 of a national center of biotechnology information (NCBI). An endothelial cell is a main target of the HBP and plays an important role in a mechanism of capillary leakage.

In the related art, the level of the HBP has been shown to correlate with certain diseases, and may be used for predicting the risk of some diseases. For example, a HBP of a patient suffering a bacterial meningitis significantly rises, but the level of the HBP in the viral meningitis is comparable to that in normal people (CN103250054B); the level of the HBP is associated with urinary tract infections, but there is only a bacterial infection (CN103380379B); and the level of the HBP is also associated with sepsis, but only bacterial, fungal, and parasitic infections are involved (CN101687023B). All of the above prior art seems to show that the HBP is not suitable for determining a viral infectious inflammation.

Inventors finds for the first time that the level of the HBP rises in a patient suffering a coronavirus disease (COVID-19). The inventors finds that the level of the HBP of a patient suffering respiratory failure caused by COVID-19 infection significantly rises compared with a patient suffering respiratory failure caused by non-infection. Moreover, the level of the HBP of the patient suffering respiratory failure caused by infection is changed in a process of disease remission, and changes of rise in the level of the HBP correspond to subsequent disease progression, which indicates that changes in the level of the HBP may be used as an early warning indicator of disease relapse in a remission period of a severe patient suffering a COVID-19. The inventors further measures, analyzes and compares correlation between the HBP indicator and an inflammatory indicator, a coagulation indicator, a blood gas indicator and a pulmonary exudation level, a myocardial indicator and a liver and kidney function indicator, and find that the HBP indicator has great correlation with the commonly used clinical indicators, and changes in the level of the HBP indicator are basically 5 days earlier than the indicators, which indicates that the HBP indicator is a preferred clinical reference biomarker for predicting progression of a pathogenic condition of the patient suffering a COVID-19, especially a severe patient suffering a COVID-19. The change of the HBP indicator may be used for predicting relapse of persistent inflammation and hypoxia-induced multi-organ failure, and may perform prediction to early take early intervention means compared with other clinical indicators, so as to prevent deterioration of the pathogenic condition.

More specifically, the inventors find for the first time that the level of the HBP has an important clinical value in a viral complex inflammatory pathway for the COVID-19. According to the invention, a method for predicting a risk of developing inflammation, especially multiple organ failure, in a patient suffering a COVID-19 is provided. The method of the present application may be used for early warning and prognostic determination of a severity of a pathogenic condition of the patient suffering a COVID-19, a use of the method of the present application is not influenced by a negative or positive nucleic acid test of the patient, and thus has a value difficult to replace in predicting the risk of deterioration in a remission period of the patient suffering a COVID-19 who seem to be getting better and relax their vigilance when nucleic acid tests turn negative. Early warning and prognostic determination of a severity of a pathogenic condition of the patient suffering a COVID-19 by the method of the present application may be used for guiding an appropriate treatment solution, which is of great significance to improve prognosis of the patient suffering a COVID-19, especially the severe patient suffering a COVID-19.

The term "deterioration" of the pathogenic condition of the patient of the present application includes situations of relapse of the patient in a remission period and aggravation of the patient in a progression period.

An actual specific use solution of the present application may include: testing the level of a HBP at least two times before and after the patient suffering a COVID-19, where an interval of two tests may be up to half a day, 1 day, 2 days, 3 days, 4 days, 5 days, 6 days, 7 days, 8 days, 9 days, or 10 days. When the level of the HBP between the two tests rises by at least 5 ng/ml, 10 ng/ml, 15 ng/ml, 25 ng/ml, 50 ng/ml, 75 ng/ml, 100 ng/ml, 125 ng/ml, 150 ng/ml, 175 ng/ml, 200 ng/ml, 250 ng/ml, 300 ng/ml, 350 ng/ml, 400 ng/ml, or 500 ng/ml, it is determined that the risk of deterioration of the pathogenic condition of the patient is high.

The present application mainly relates to a clinical use for human patients, but the present application may also be used for a clinical use for non-human animals.

The present application relates to measurement of the level of the HBP of the patient suffering a COVID-19. The level of the HBP is usually measured in an ex vivo sample obtained from the patient. The sample usually includes a body fluid of the patient. The body fluid sample may be a blood, plasma, serum, urine, cerebrospinal fluid or joint fluid sample. The sample is preferably a plasma sample. Standard methods known in the art, such as an immunological measurement method, may all be used for measuring the level of the HBP. The immunological measurement method includes a fluorescence immunochromatography and an enzyme-linked immunoassay. Other measurement methods, such as high performance liquid chromatography separation and fluorescence detection, may also be used for measuring the level of the HBP.

The present application further relates to a diagnostic kit for measuring the level of a HBP in a patient suffering a COVID-19, to determine whether the patient has a risk of deterioration of a pathogenic condition. Preferably, the kit usually includes one or more antibodies that specifically bind the HBP. For example, the kit may include a monoclonal antibody, a polyclonal antibody, a single-chain antibody, a chimeric antibody, a complementary determining region (CDR)-grafted antibody, or a humanized antibody. The antibody may be an intact immunoglobulin molecule or a fragment of the immunoglobulin molecule, such as a Fab fragment, a F(ab')₂ fragment and a Fv fragment. If more than one antibody exist, the antibody preferably has different non-overlapping determinants, so as to enable the non-overlapping determinants to bind the HBP simultaneously.

The kit may additionally include one or more other reagents or instruments that may execute any one of implementation solutions of the method mentioned above. The reagents or instruments include one or more of the following: a suitable buffer solution (aqueous solution), a tool separating a HBP from a sample, a tool (such as a vessel or an instrument including a needle) obtaining a sample from a patient, or a support including a well on which a quantitative reaction may be carried out. The kit may optionally include a specification enabling the kit to be used in the method of the present application or details regarding which individuals the method may be performed.

### BRIEF DESCRIPTION OF DRAWINGS

Fig. 1 shows changes in the level of a heparin-binding protein (HBP) correlated with a degree of a coronavirus disease (COVID-19).
Fig. 2 shows a comparison of change trends of a high resolution computed tomography (HRCT) and a chest posterior-anterior (PA)&lateral (LAT) of a critical patient suffering a COVID-19.
Fig. 3 shows correlation analysis between a gas indicator and the HBP. (PA-aDO2: arterial-alveolar oxygen tension difference; Qsp: intrapulmonary shunt volume; ABE: actual base excess; SBE: standard base excess; Spiro index: respiratory index; and OI: oxygenation index).
Fig. 4 shows a longitudinal trend of changes in a myocardial test indicator of a patient suffering a COVID-19 and a relation between the myocardial test indicator and the HBP. (AST: aspartate amino transferase; CK: creatine kinase; CK-MB: creatine kinase isoenzyme; cTnI: cardiac troponin I; and Mb: myoglobin).
Fig. 5 shows a trend and cross-correlation function (CCF) analysis of a liver and kidney function indicator.

### DETAILED DESCRIPTION OF THE EMBODIMENTS

### 1. Experimental method

### 1.1 Grouping standard

12 patients suffering infectious critical respiratory failure were included in an intensive care unit (ICU) of First Affiliated Hospital of Guangzhou Medical University according to Global Surveillance for human infection with coronavirus disease (COVID-19), Interim guidance, 20 March 2020. An exclusion standard was history of a chronic liver or a kidney disease. According to a negative time point tested for COVID-19 ribonucleic acid (RNA), the beginning of a remission stage of a patient was defined, and moreover, it was necessary to satisfy characteristics of deterioration of a pathogenic condition during follow-up. Sputum and urine bacteria or fungi were tested negatively, and nosocomial secondary infection was excluded. Age-and gender-matched patients suffering non-infection-induced respiratory failure were used as control groups (n=15). Clinical information for all participants was available, and the level of a heparin-binding protein (HBP) was tested for each participant.

A course of the severe patient suffering a COVID-19 was divided into two stages: 1. "admission stage", which was defined as a time point at which an acute disease occurs; and 2. "remission period" (negative COVID-19 RNA). A starting point was determined by 3 clinicians according to an overall clinical state of the patient, and a pneumonia severity index was evaluated (PSI; data not shown). In order to clearly distinguish different periods, specific stages of the patient were indicated in all data. The study lasted 125 days and was approved by Ethics Committee of First Affiliated Hospital of Guangzhou Medical University (2020-77).

### 1.2 HBP quantitative test

A sodium citrate anticoagulant (1: 9) plasma sample was used for test. During plasma separation, it was noted that any white blood cell may not be inhaled, so as to prevent the white blood cell from releasing a high level of the HBP. A Jet-iStar 3000 full-automatic immunoanalyzer (Zhonghan Shengtai Biotechnology Co., Ltd., Zhejiang, China) was used for testing the 50µl plasma sample, and the level of the HBP was tested after 18 min incubation (dry fluorescence immunoassay).

### 1.3 Imaging test

Imaging examination was carried out on pulmonary lesions, to obtain analysis of a posteroanterior position, an oblique position, and a lateral position (posterior-anterior (PA)&lateral (LAT)) of a chest and quantitative high resolution computed tomography (HRCT). In chest PA&LAT examination, it should be noted that it was ensured that image blurring caused by breathing movement of the patient may not be caused, and therefore fast exposure (0.08s) was used. A centerline of a PA test plate was aligned with a dorsal 5th thoracic vertebra, and a centerline of a LAT test plate was aligned with a lateral chest wall of the 5th thoracic vertebra. A cassette needed to have an upper end higher than an upper portion of a contralateral shoulder by 3 cm and a lower end covering a costophrenic angle region. Main evaluation included a pulmonary texture, a pulmonary consolidation shadow area, a cardiac shadow area, and a costophrenic angle. A percentage of a lesion area out accounting for a total lung area was used as an indicator to evaluate a lung involved area. The HRCT had a layer thickness set to 1.0 mm. A quantitative analysis system was used for evaluating areas and shapes of a mutation shadow, a patch shadow, and a fiber-strip-shaped shadow. In addition, an abdominal HRCT image was further collected, so as to evaluate an organ function (e.g., abnormally low or high density) during a study.

### 1.4 Statistical analysis

Continuous variables were represented as medians and an interquartile range (IQR). Non-parametric test (Mann-Whitney test) was used for differential analysis between groups. P<0.05 was considered to be statistically significant. A cross-correlation function (CCF) was used for verifying correlation between different indicators and the level of the HBP. Stage relations between trends of different indexes and a trend of the HBP were verified by determining a maximum time delay in CCF values. R (Bell Laboratory version 4.0.0), GraphPad Prism 8.0.2 (GraphPad Software, San Diego, California, USA) and IBM SPSS (Statistics for Windows Version 22.0, IBM, Chicago, Illinois, USA) were used for analyzing data and plotting graphs.

### 2. Experimental result

### 2.1 Patient characteristics

**Table 1 Overall information of participants**

| | Respiratory failure | Severe COVID-19 | P value |
|---|---|---|---|
| N | 15 | 12 | --- |
| Age, years | 56.00 (37.00, 65.00) | 59.50 (51.25, 71.00) | 0.187 |
| Gender, male/female | 9/6 | 8/4 | 0.816 |
| HBP, ng/ml | 12.58 (5.90, 29.85) | 109.10(50.57, 186.00) | 0.001 |
| Alanine aminotransferase (ALT), U/L | 23.77(8.19, 36.47) | 20.25 (12.95, 33.75) | 0.076 |
| Aspartate amino transferase (AST), U/L | 31.60(30.10, 40.30) | 38.75(28.28, 60.78) | 0.002 |
| Blood urea nitrogen (BUN), mg/dl | 6.18(5.43, 7.20) | 9.80 (6.80, 13.40) | 0.002 |
| Creatinine (Cr), umol/ml | 67.10(54.39, 91.01) | 76.40 (55.60, 103.50) | 0.042 |
| CRP, mg/L | 0.13 (0.05, 0.24) | 9.27 (5.66, 15.10) | 0.001 |
| D-dimer | 811 (159.8, 3500.00) | 3543 (1914, 6222) | 0.001 |
| PCT, ug/L | 0.11 (0.05, 0.17) | 0.38 (0.16, 0.95) | 0.001 |
| K, mmol/L | 4.27(4.10, 4.62) | 4.17 (3.93, 4.51) | 0.072 |
| Na, mmol/L | 135.20(135.50, 140.10) | 139.50(136.10, 143.60) | 0.295 |
| Cl, mmol/L | 97.10(94.33, 104.13 | 104.60(99.00, 109.20) | 0.367 |
| Ca, mmol/L | 2.24(2.20, 2.51) | 2.30(2.17, 2.40) | 0.143 |
| White blood cell, 10^9/L | 5.13(4.77, 7.31) | 9.34(7.10, 11.30) | 0.001 |
| Polymorphonuclearleukocyte , 10^9/L | 2.31(1.94, 4.00) | 6.70(5.10, 8.70) | 0.001 |
| Lymphocyte, 10^9/L | 1.53(0.97, 1.66) | 0.90(0.60, 1.40) | 0.034 |
| REed blood cell, 10^12/L | 4.31(3.44, 5.19) | 2.96(2.66, 3.32) | 0.001 |
| Blood platelet, 10^9/L | 154.00(117.00, 203.00) | 165.00(108.00, 219.00) | 0.470 |

The level of the HBP of the critical patient suffering a COVID-19 was significantly higher than that of other patients suffering respiratory failure (Table 1). CRP and PCT of the patient suffering a COVID-19 were also high. Counts of the white blood cell and the polymorphonuclearleukocyte of the patient suffering a COVID-19 were also higher, but a count of lymphocyte was lower. The level of D-dimer of the patient suffering a COVID-19 significantly rose, which indicated that although the patient did not have hypotensive shock and did not satisfy a diagnostic standard of disseminated intravascular coagulation (DIC), there were still extensive microcirculation disorders.

### 2.2. Comparative analysis of HBP and multi-system indicators

### 2.2.1 Change trend of HBP and correlation analysis with other inflammatory indicators

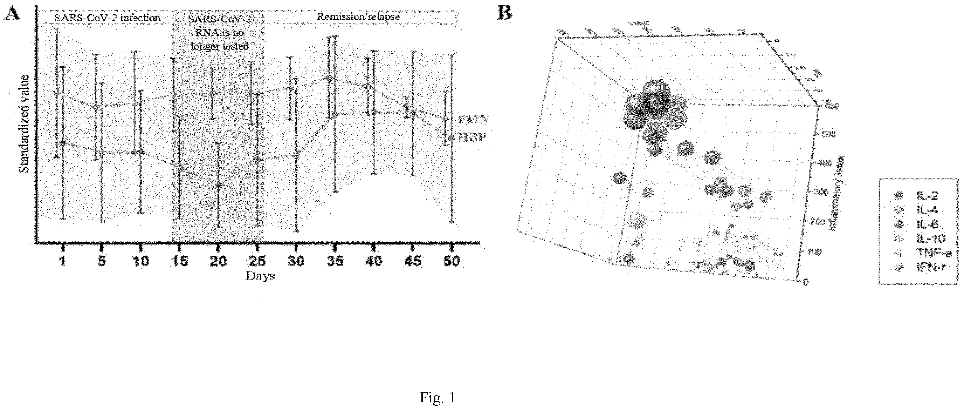

A change of the level of the HBP correlated with a degree of the COVID-19 was as shown in Fig. 1.

A. Comparison of longitudinal change trend of HBP and PMN during hospitalization. In Fig. 1, COVID-19 infection represented a remission period of the COVID-19, but RNA test was positive. The remission period/relapse period referred to a period when a virus in the remission period turns negative, during which the pathogenic condition of the patient was still deteriorated anew.

B. Further analysis of remission period/relapse period. Six inflammatory indicators were compared after standardization. The larger a sphere was, the higher the level was. A yellow line represented a distance between the sphere and a coordinate plane.

Trend comparison of the HBP and the PMN found (correlation between the HBP and the PMN was significant) (Fig. 1A) that the level of the HBP in the remission period had a decline trend (a left side of a chart). Even if COVID-19 RNA had turned negative, the level of the HBP rose anew, which was parallel to the degree of deterioration of the pathogenic condition of the patient. The level of the HBP reached a peak value on the 10th day of disease relapse (35th day in the figure), was maintained for 10 days at a peak period, and then was declined on the 50th day. PMN also had a similar change trend, but an increase range was not as obvious as the HBP, and a later decline range was large. The level of the HBP was significantly positively correlated with CRP and PCT (r=0.463, 0.497, P<0.001).

The HPB was compared with an inflammatory indicator (Fig. 1B) formed by IL-2, IL-4, IL-6, IL-10, a tumor necrosis factor α (TNF-α) and interferon-γ (IFN-γ), increase in IL-6 was the most significant in a course. The variables were further projected onto the inflammatory indicator and the plane of the HBP, so as to intuitively display a linear relation, and it may be seen that IL-6 was significantly positively correlated with the HBP (r=0.693, P<0.001), and the HBP, IL-6 and course time were also significantly positively correlated (P<0.001).

### 2.2.2 Analysis of coagulation indicator

**Table 2. Coagulation indicator**

| | COVID-19 infection remission period | &HBP P | Relapse period | &HBP P | *Difference | *P |
|---|---|---|---|---|---|---|
| PT | 14.60(14.20, 15.10) | 0.333 | 15.00(14.40, 15.60) | 0.022 | 0.40 | 0.134 |
| PA | 83.00(76.00, 88.00) | 0.251 | 78.00(72.00, 85.00) | 0.021 | -5.00 | 0.145 |
| Fibrinogen | 2.98(2.44, 4.05) | 0.438 | 2.50(2.04, 3.30) | 0.849 | -0.48 | 0.338 |
| APTT | 45.50(40.40, 49.80) | 0.025 | 48.17(41.70, 54.80) | 0.036 | 0.40 | 0.036 |
| TT | 17.60(16.70, 1950) | 0.675 | 17.50(16.60, 19.30) | 0.407 | -0.10 | 0.946 |
| D-dimer | 2439.00(791.00, 6667.00) | 0.025 | 4682.00(604.50, 5988.00) | 0.017 | 2243.00 | 0.001 |

The COVID-19 infection remission period represented the patient suffering a positive COVID-19 test (which was marked as COVID-19 infection in Fig. 1A). When the patient was negative for the COVID-19, relapse was the same as remission/relapse in Fig. 1A.

PT: prothrombin time; PA: prothrombin activity; APTT: activated partial thromboplastin time; TT: thrombin time; &HBP: HBP relative to a left column. Comparison of remission and relapse of COVID-19 infection

reflected that intrinsic coagulation was longer in the relapse period than in the remission period, and there was a significant difference between the relapse period and the remission period (Table 2). D-dimer obviously rose in the COVID-19 negative relapse period and was positively correlated with the HBP. However, comprehensive clinical indicators and manifestations of the patient have not reached a diagnostic criteria of the DIC.

### 2.2.3 Correlation between blood gas indicator and pulmonary exudation level

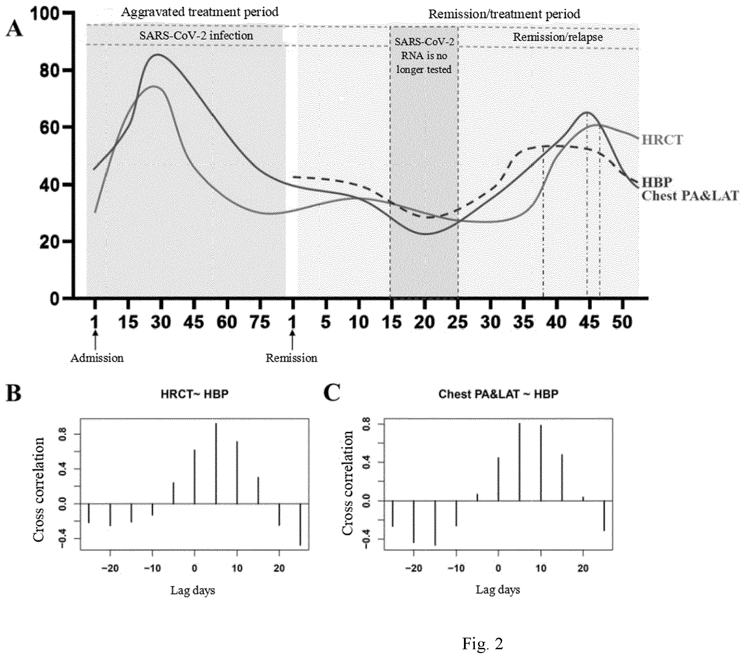

The change trend of a HRCT and a chest PA&LAT of the critical patient suffering a COVID-19 was shown in Fig. 2.
(A) Y-axis was a percentage of a lung area having exudative lesions in the HRCT and the chest PA&LAT. The figure showed time when the three indicators reach a peak value.
(B) CCF analysis of HRCT and HBP. Correlation between the HRCT and the HBP was the highest, and the HRCT lagged by 5d (r=0.92, P<0.05).
(C) CCF analysis of chest PA&LAT and HBP. Correlation between the chest PA&LAT and the HBP was also the highest at 5 days (r=0.804, P<0.05).

Lung effusion change data of the HRCT and the chest PA&LAT was extracted at admission, and was compared with a subsequent HBP track. In order to display a height and time of the peak value intuitively, a curve is smoothed (Fig. 2). It was suggested that the HBP rose earlier around 35-40 days, and the peak values of the HRCT and the chest PA&LAT appeared around 45 days. By computing a correlation function between the HRCT and the chest PA&LAT (Figs. 2B and 2C), it was found that the HRCT and the chest PA&LAT had the strongest correlation with the HBP when lag days were equal to 5. Therefore, the HBP was increased and reached the peak value around 5 days before the HRCT and the chest PA&LAT.

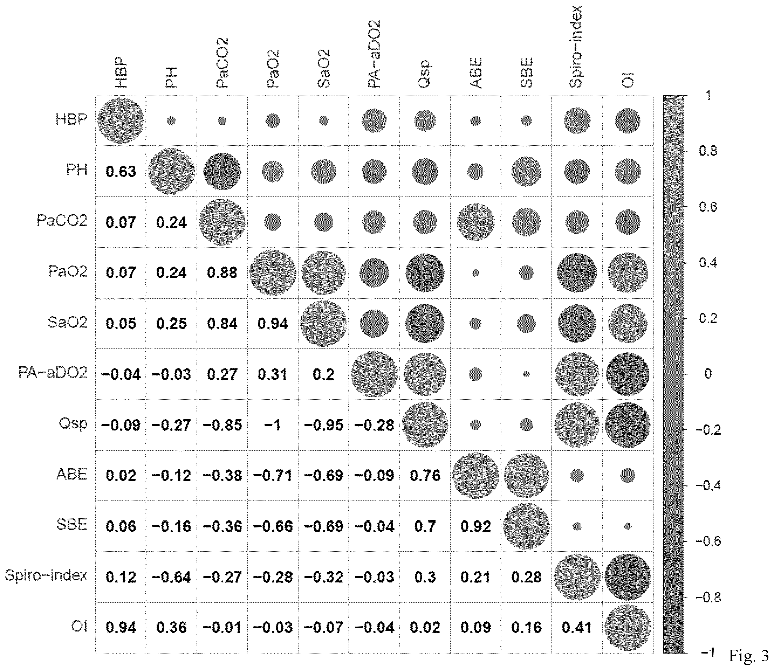

Correlation analysis between the gas indicator and the HBP of the patient was as shown in Fig. 3. Analysis showed that the HBP was correlated with PA-aDO2, Qsp, Spiro-Index and OI.

### 2.2.4 Function evaluation of extrapulmonary organs of patient suffering COVID-19

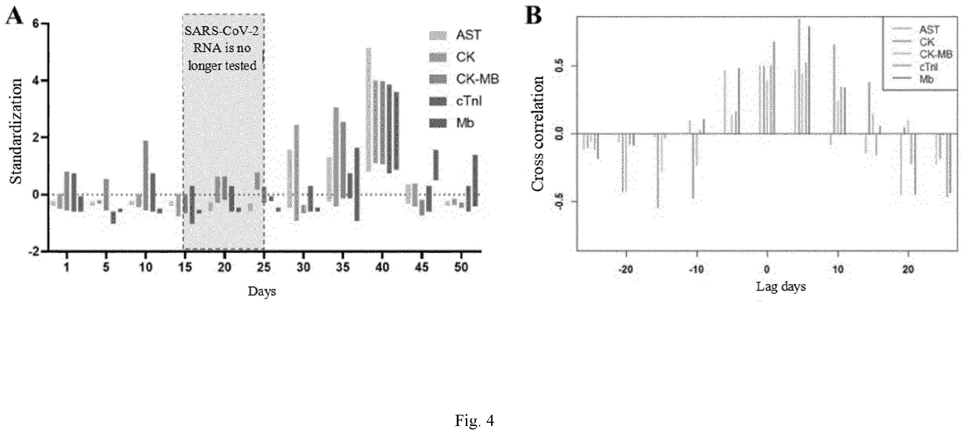

Function evaluation of extrapulmonary organs of the patient was as shown in Fig. 4, which showed a longitudinal change trend of a myocardial test indicator of the patient suffering a COVID-19 and a relation between the myocardial test indicator and the HBP. Correlation between the myocardial test indicator and the HBP was the highest when lag days were 5 (AST: r=0.499, p=0.1179; CK: r=0.848, p<0.1983; CK-MB: r=0.438, p=0.1983; cTnI: r=0.524, p=0.0982; and MB: r=0.789, p<0.05).

**Table 3 Myocardial indicator**

| | COVID-19 infection remission period | &HBP P | Relapse period | &HBP P | *Difference | *P |
|---|---|---|---|---|---|---|
| AST | 38.50(29.13, 51.68) | 0.024 | 49.25(37.55, 65.53) | 0.031 | 10.75 | 0.001 |
| CK | 53.00(33.85, 80.70) | 0.046 | 98.15(22.83, 214.90) | 0.007 | 45.15 | 0.001 |
| CK-MB | 7.00(6.00, 9.00) | 0.409 | 8.50(5.00, 10.00) | 0.361 | 0.5 | 0.027 |
| cTnI | 0.02(0.01, 0.06) | 0.017 | 0.05(0.02, 0.07) | 0.024 | 0.03 | 0.029 |
| Mb | 69.45(42.63, 158.10) | 0.031 | 186.30(125.90, 312.20) | 0.004 | 116.85 | 0.001 |

AST: aspartate amino transferase; CK: creatine kinase; CK-MB: creatine kinase-MB; CTnI: cardiac troponin I; and Mb: myoglobin. HBP: comparison of the HBP with a left column. COVID-19 infection remission and relapse were compared.

After a COVID-19 RNA test was negative, the overall level of serum AST, CK, CK-MB, cTnI and Mb rose in 35-45 days after the patient suffering a COVID-19 relapsed (Table 3, Fig. 4A). During this period, Pro-BNP of the patient was 2479 (603.4, 3379) ng/mL, which was obviously higher than a normal value. Urine amount of the patient was not reduced obviously. Imaging showed that 1 patient had pericardial effusion, but no cardiac shadow enlargement or cardiovascular function abnormality. Compared with the change of the level of the HBP (the peak value is 35-40 days), the change of the above 5 indicators had a certain lag. After the correlation coefficient (Fig. 4B) was computed, it was found that correlation with the HBP was the strongest anew when the HBP was lagged for 5 days, and the HBP reached the peak value 5 days earlier than AST, CK, CK-MB, cTnI and MB.

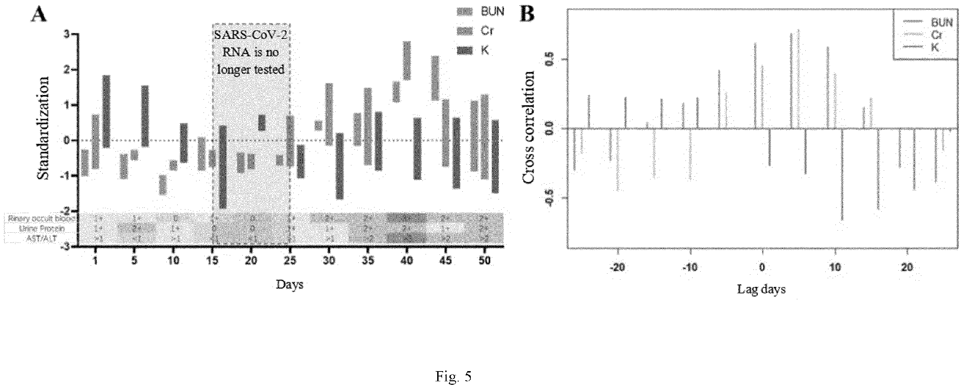

The trend and CCF analysis of the liver and kidney function indicator of the patient were as shown in Fig. 5. BUN and Cr were significantly positively correlated with the HBP. Correlation was the highest by 5 days (BUN: r=0.684, p<0.05; and Cr: r=0.714, p<0.05) anew, but there was no obvious positive correlation between K⁺ and the HBP.

**Table 4 Test indicator of kidney function**

| | COVID-19 infection remission period | &HBP P | Relapse period | &HBP P | *Difference | *P |
|---|---|---|---|---|---|---|
| BUN | 10.45(7.38, 13.93) | 0.001 | 11.60(8.35, 12.25) | 0.006 | 1.15 | 0.001 |
| Cr | 76.50(48.68, 99.35) | 0.034 | 90.15(54.55, 146.40) | 0.015 | 13.65 | 0.001 |
| K⁺ | 4.14(3.88, 4.54) | 0.902 | 4.19(4.02, 4.38) | 0.712 | 0.05 | 0.884 |

### BUN: blood urea nitrogen

BUN, Cr and K were selected as indicators reflecting a kidney function. BUN and Cr showed an obvious rise trend in the relapse period, and K⁺ showed no obvious rise trend (Table 4, Fig. 5A). During this period, an abdominal CT showed no obvious abnormality in the kidney. CCF analysis (Fig. 5B) showed that BUN and Cr were significantly positively correlated with the HBP, which indicated that the change of BUN and Cr reflected the change of the HBP, which had lag time of 5 days. For K⁺, the value was stable and there was no obvious rise or a peak value, and therefore correlation between K⁺ and the HBP was extremely weak.

An AST/ALT ratio, as an indicator reflecting liver damage, showed an rise trend from 35d, and reached a peak value greater than 3 at 40d, which suggested that liver cell destruction was serious. However, during this period, the abdominal CT showed no new abnormal low-density lesions in the liver.

### 3. Analysis of example of the present application

In an example of the present application, pathogenic conditions of some of the severe patients suffering a COVID-19 in the remission period were deteriorated anew, and some patients had multiple organ dysfunction, possibly because systemic inflammatory responses of elderly patients were strong, and patients suffering weakened adaptive immunity and malnutrition may have severe clinical manifestations and be more likely to have nucleic acid positive recovery due to persistence of non-infection-correlated secondary inflammation, which was one of main reasons for sudden decline of the pathogenic condition and even death in the remission period, and was equivalent to the risk of sudden cardiac death caused by the COVID-19. Inventors speculated that this may be caused by persistent inflammation caused by imbalance of immune functions of a body caused by the COVID-19. Pathogens and fragments of the pathogens released after cell tissue destruction were recognized as foreign matters by the body. In the case of aggravation of the pathogenic condition, immune function imbalance was prone to occur. Overactivation of innate immunity aggravated damage to tissues and organs, thereby forming a vicious circle.

In the example of the present application, the change of the level of the HBP and PMN was consistent with correlation of deterioration of the pathogenic condition in the remission period of the disease, both of which started to rise in 30 days and reached the peak in 35 days. In the example of the present application, correlation analysis of six measured inflammatory factors showed that the level of IL-6 significantly rose, and was correlated with the HBP, and the two factors were parallel to the progression of the disease over time. The level of IL-6 rose at an early stage of an inflammatory storm, and then CRP, PCT and an amyloid protein rose, which were all positively correlated with the progression of inflammation and apoptosis inhibition of PMN.

In addition, according to the example of the present application, dynamic comparison of pulmonary exudative lesions correlated to the HBP, the HRCT and the chest PA&LAT found that the HBP rose 5 days earlier than the progression of pulmonary lesions, and was significantly correlated to the change of a lung effusion area. Therefore, from the perspective of imaging, the HBP may be used as a prediction indicator of pulmonary lesion progression. Therefore, compared with the commonly used inflammatory factors CRP, PCT and amyloid protein, the HBP may not only reflect the degree of inflammation having high sensitivity, but also participate in a mechanism of inflammation progression. In addition, the HBP had certain anti-inflammatory and antibacterial effects, and had advantages in evaluating the severe patients suffering a COVID-19.

According to an experiment of the present application, it may be found that severe hypoxemia commonly existed in the severe patients suffering a COVID-19, and acute diffuse respiratory distress syndrome was a main factor leading to poor prognosis. According to the experiment in the example of the present application, it may be found that the level of the HBP was significantly correlated with an intrapulmonary shunt volume, an arterial-alveolar oxygen tension difference, a respiratory index and an oxygenation index. Under the influence of capillary leakage syndrome and vascular endothelial cell damage caused by the HBP, a respiratory membrane and pulmonary blood flow perfusion were influenced, which ultimately led to the decline of a pulmonary ventilation function. Therefore, in the COVID-19, the HBP was closely correlated with pulmonary ventilation and parallel to the degree of hypoxia. The closely correlated result also highlighted the relation between the HBP and a mechanism of intrapulmonary shunt abnormalities.

According to the example of the present application, markers of myocardial damage showed a rise trend in the relapse period, and 1 patient had pericardial effusion. According to the example of the present application, the peak value of AST/ALT was greater than 3. Moreover, liver damage was more common in severe patients than in light patients suffering a COVID-19, and it was reported that AST significantly rose in severe patients. In the relapse period, hepatocellular mitochondria was damaged, a large amount of AST was released, and the ratio of AST/ALT significantly rose. The ratio greater than 3 represented serious damage of liver tissue. BUN and Cr rose, and urine occult blood and urine protein were both positive. 2 severe patients were diagnosed as acute kidney damage. Renal perfusion is affected by hypoxia and systemic inflammation, leading to glomerular filtration dysfunction. However, the study did not find significant reduction in urine amount or electrolyte imbalance (such as K⁺).

In the severe patient suffering a COVID-19, the remission period after the virus RNA test turned negative should not be taken lightly. The multiple organ failure caused by a persistent state of inflammation and imbalance of an immune function in the remission period still caused sudden deterioration of the pathogenic condition. According to previous studies, no viral inclusion bodies were tested in the heart, liver and kidney, which indicated that direct extrapulmonary infection of the virus was not the main reason of deterioration. According to the example of the present application, even under the condition that the inflammation was controlled, some indicators reflecting the degree of organ damage was also not reduced.

Therefore, according to the above example of the present application, the HBP played a key role in the inflammatory response of the severe patients suffering a COVID-19, especially in disease relapse in the viral remission period. Longitudinal analysis showed that the HBP rose around 5 days before multiple organ dysfunction and pulmonary imaging pathological features appeared. Therefore, it was deemed that monitoring of the HBP may be a useful prediction indicator of early multiple organ damage in sequelae of COVID-19 infection. Early intervention based on monitoring of the HBP may improve prognosis of the severe patient suffering a COVID-19.

What are described above are merely different specific examples of the present application but not intended to limit the present application, and any modifications, equivalent replacements, improvements, etc. made within the spirit and principles of the present application should fall within the scope of protection of the present application.

## Claims

1. A use of a heparin-binding protein (HBP) in preparation of a kit used in a method for predicting early warning and prognostic determination of a severity of a pathogenic condition of a patient suffering a coronavirus disease (COVID-19), wherein the method comprises: testing a change in a concentration of the HBP of the patient suffering a COVID-19.

2. The use according to claim 1, wherein the change in the concentration of the HBP of the patient suffering a COVID-19 is tested by testing an ex vivo sample from the patient.

3. The use according to claim 2, wherein the ex vivo sample is plasma.

4. The use according to claim 1, wherein the patient suffering a COVID-19 is a severe patient or critical patient.

5. The use according to claim 1, wherein the patient suffering a COVID-19 is in a remission period.

6. The use according to claim 1, wherein the method for predicting early warning and prognostic determination of a severity of a pathogenic condition of a patient suffering a COVID-19 comprises: determining whether the concentration of the HBP of the patient before and after at least two tests on a time series rises.

7. The use according to claim 6, wherein the concentration of the HBP in the two tests rises by at least 10 ng/ml.

8. The use according to claim 6, wherein the concentration of the HBP in the two tests rises by at least 50 ng/ml.

9. The use according to claim 6, wherein the concentration of the HBP in the two tests rises by at least 100 ng/ml.

10. The use according to claim 1, wherein the patient further comprises a non-human animal.
